# EUROPEAN PATENT SPECIFICATION

(11) **EP 2 579 845 B1**
(45) Date of publication and mention of the grant of the patent: **29.04.2020**
(21) Application number: 11739184.7
(22) Date of filing: 13.06.2011
(51) Int. Cl.: A61K 9/107, A61K 47/10, A61K 47/14

(54) **OPHTHALMIC COMPOSITIONS FOR THE ADMINISTRATION OF LIPOSOLUBLE ACITVE INGREDIENTS**
OPHTHALMISCHE ZUSAMMENSETZUNGEN ZUR VERABREICHUNG VON FETTLÖSLICHEN WIRKSTOFFEN
COMPOSITIONS OPHTALMIQUES POUR L'ADMINISTRATION D'INGRÉDIENTS ACTIFS LIPOSOLUBLES

(30) Priority: 11.06.2010 IT RM20100322
(43) Date of publication of application: 17.04.2013
(73) Proprietor: Medivis S.R.L., 95127 Catania (CT) (IT)
(72) Inventor: MANGIAFICO, Sergio, I-95127 Catania (IT); ALEO, Danilo, I-95127 Catania (IT); SAITA, Maria Grazia Antonietta, I-95127 Catania (IT); CRO, Melina, I-95127 Catania (IT)
(74) Representative: Banchetti, Marina
(86) International application number: PCT/IT2011/000196
(87) International publication number: WO 2011/154985

(56) References cited:
- EP-A1- 1 681 059
- WO-A1-03/032949
- WO-A1-2011/063367
- WO-A2-2010/045292
- US-A1- 2004 191 284
- US-B1- 6 479 540
- PERLMAN ET AL: "Development of a self-emulsifying formulation that reduces the food effect for torcetrapib", INTERNATIONAL JOURNAL OF PHARMACEUTICS, ELSEVIER BV, NL, vol. 351, no. 1-2, 5 February 2008 (2008-02-05), pages 15-22, XP022455513, ISSN: 0378-5173, DOI: DOI:10.1016/J.IJPHARM.2007.09.015
- VANDAMME TH F: "Microemulsions as ocular drug delivery systems: recent developments and future challenges.", PROGRESS IN RETINAL AND EYE RESEARCH JAN 2002 LNKD- PUBMED:11906809, vol. 21, no. 1, January 2002 (2002-01), pages 15-34, XP002630484, ISSN: 1350-9462

## Description

The present invention concerns topical ophthalmic compositions for the administration of liposoluble or poorly water-soluble active ingredients. More particularly, the invention concerns ophthalmic emulsions suitable for the administration of lipophilic or scarcely water-soluble active ingredients on the ocular surface, the said ophthalmic emulsions consisting of microemulsions or self-emulsifying systems characterized by a high stability and a particularly low surfactant content.

In the latest years the pharmaceutical research has focused its interest on highly liposoluble or scarcely water-soluble molecules. It is estimated that about 40% of the recently developed new active ingredients are poorly soluble in water or lipophilic. These molecules have found a use not only for the treatment of pathologies that are treated through the oral or systemic routes of administration but also in the treatment of pathologies affecting the anterior and posterior chamber of the eye, such as glaucoma, uveites, keratites and conjunctivites, dry-eye syndrome or lachrimal film disorders, age-related macular degeneration, retinites, autoimmune disorders and so on.

The formulations for topical ophthalmic use containing lipophilic (or sparingly water-soluble) active ingredients presently in use consist of oily solutions, lotions, ointments, gels and particular delivery systems, such as intraocular inserts. None of such systems, however, offers a good compliance by the patients, as the administration of these preparations may be troublesome, or may generate discomfort or a blurred vision sensation, or even irritation and burning on the ocular surface. Further, many of such systems are also scarcely efficient from a clinical point of view, in that they do not warrant a high absorption of the medicament at the site of action.

On the other hand, the conventional aqueous-based ophthalmic compositions have to cope with the problem of a low residence time at the corneal level, which results from the tears drainage. In spite of the fact that such drawback has been partially overcome by increasing the viscosity of the aqueous systems, achieving a suitable bioavailability of the active ingredients applied on the eye surface also in the inner structures of the eye is still an open challenge.

In such a framework, the oil-in-water (o/w) emulsions, specifically lipidic emulsions, used since a long time for parenteral applications, are being increasingly studied also in ophthalmology, with the aim of formulating lipophilic active ingredients and improving their ocular bioavailability. The success of some products recently marketed in such form, such as, for instance, cyclosporine A in ophthalmic emulsion (Restasis®), or the preparation based on medium chain fatty acids (MCT, medium chain triglycerides) for the treatment of dry eye (Lipimix®), or the steroidal drug difluprednate in ophthalmic emulsion (Durezol®), fostered further research in this field. The high biodegradability, the submicron size of the dispersed phase and the resulting connected possibility of sterilizing simply by filtration, as well as the anticipated increase in bioavailability of the carried drug, make this system a very promising instrument of the formulation of topic ophthalmic compositions.

As it is known, an emulsions is a system formed by two immiscible liquid phases intimately admixed and dispersed one into the other, obtained through an input of energy from the outside, for instance through mechanical stirring and/or heating. The thermodynamic function defining an emulsion is **W** = γ **ΔA,** wherein W is the necessary work for the dispersion, γ is the value for the surface tension and ΔA is the surface area of the system. Given the difference in attractive interaction between the various molecules of the two liquid phases, an interfacial tension is originated in any point where the two liquids are in contact (interface), and the particles of the dispersed phase tend to aggregate in order to reduce the contact surface area (ΔA), thus reducing the connected work (a phenomenon leading to phase separation).

It is also known that the interfacial tension may be significantly lowered by adding amphiphilic molecules or surfactants soluble in at least one of the two phases of the emulsion. Thus, the addition of a suitable surfactant, the molecules of which go to place themselves at the interface between aqueous phase and oily phase, can increase the kinetic stability of the emulsion, thereby avoiding the phase separation, or at least slowing down its development.

The systems described above more properly correspond to **classic emulsions,** defined as biphasic systems consisting of an oily and an aqueous phase kept together by an emulsifier (surfactant), which increases and prolongs the physical stability of the system. A co-emulsifier is almost always coupled to the main emulsifier, resulting in a further increase of the emulsion stability. The described category also includes the so-called nanoemulsions (or sub-micronic emulsions, SMEs) which differ from the previous emulsions only in the finer sizes of the dispersed particles. The latter can be defined as oil-in-water emulsions having average diameter of the dispersed droplets varying from 50 to 1000 nm (typically, the average size is comprised between 100 and 500 nm).

These systems are *metastable* systems, which may be stable for months or years before undergoing natural separation phenomena. As pointed out, such systems need an exterior force in order to form, for instance heating of the two phases, treatment with high-speed mixers, high pressure homogeneization. It is to be noted that some systems require, in order to be formed, the application of two or even all of the three cited methods.

By way of example, lipid emulsions belonging to the class of classic emulsions/nanoemulsions for use as carrier of lipophilic active ingredients are described in the document EP 0391369 (Yissum Resarch Development Company of the Hebrew University of Jerusalem, inventors B. Simon e L. Menashe). Such compositions comprise an oily vehicle consisting of medium chain triglycerides (MCT), optionally combined with vegetal oil, in particular soybean oil, together with phospholipids (for instance lecitines, or soy phospholipids) and with surface active agents, in particular non-ionic surfactants (such as e.g. polysorbate 80 or Tween 80) and ionic surfactants (in particular cholic and deoxycholic acids). The compositions disclosed in the document are reported as being high stability emulsions formulations, and are proposed for the oral, parenteral and topic ophthalmic administration of lipophilic active ingredients.

A further example of formulation in ophthalmic emulsion for sparingly soluble active ingredient, cyclosporine A, is disclosed in the document EP 1809238 (Novagali Pharma S.A.), which proposes an alternative to the prior cited preparation, marketed by Allergan under the trademark Restasis®. Also in this case, as in the case of the Allergan product, the product is a nanoemulsion wherein medium chain tryglicerides (MCT) are used in place of the castor oil used in the earlier product as the oily phase, and a specific non-ionic surfactant, tyloxapol, is used as emulsifier in place of the couple polysorbate 80/Pemulen of the earlier product. The corresponding preparation process requires two separate homogeneizations at room temperature followed by heating at 75°C and high shear homogeneization (high shear mixing), and then by a fourth operation of high pressure homogeneization.

The most technologically advanced ophthalmic emulsion presently on the market is the product Durezol® (Sirion Therapeutics), containing 0.05% difluprednate. Also in this case, the preparation belongs to the class of classic emulsions and nanoemulsions. As reported in the corresponding scientific literature, obtaining this system requires as many as three homogeneization operations, the first two carried out at 70°C and the third one carried out by means of high pressure homogeneization. At the end of the complex preparation, particles having size comprised between 40 and 400 nm (with average size of 104.4 nm) are obtained, and the emulsion has a pH of 5.5. This emulsion contains 5% of castor oil as the oily phase and 4.0% of polysorbate 80 as emulsifier (Yamaguchi M. et al., Formulation of an ophthalmic lipid emulsion containing an anti-inflammatory steroidal drug, difluprednate, *Int. J. Pharm.* 301 (**2005**) 121-128).

A second class of biphasic systems consisting of an oily phase and an aqueous phase finely interspersed is the class referred to as "**microemulsions**" by the first authors who studied it. These authors introduced the concept of microemulsions in 1943. (Hoar T.P. e Shulman J.H., Transparent water in oil dispersions: the oleopathic hydromicelle, *Nature* 152 (**1943**) 102-103). The microemulsions are clear, isotropic and stable liquid mixtures of an oily phase and an aqueous phase, kept together by a surfactant, generally combined with a co-surfactant. The latter have the feature of forming spontaneously without intervention of any contribution from exterior forces.

Such systems are formed when a) the tension at the oil-water interface is brought to a very low level and b) the interfacial layer is maintained very flexible and fluid. These two conditions are obtained, generally, through an accurate and precise selection of the components and of their relative proportions, and through the use of a co-surfactant, which adds flexibility to the oil-water interface. Consequently, a thermodynamic optimized structure is obtained which, differently from the nanoemulsion structure, is thermodynamically stable, and requires a minimal input of energy to be formed. In this case, the work necessary to obtain the dispersion is close to zero and the system, in addition to be formed with an extremely low input of energy, has a practically unlimited stability and practically never undergoes phase separation.

Another remarkable difference between classic emulsions and microemulsions can be found in the size of the particles dispersed in the continuous phase, which are the range of 5-50 nm in microemulsions, while the size of nanoemulsions particles is typically from 50 to 500 nm.

One of the first examples of exploitation of microemulsions for the production of delivery systems for sparingly soluble active ingredients in the ophthalmic field is represented by the work of Siebenbrodt and Keipert. They studied microemulsions based on a non-ionic polymeric surfactant (poloxamer) and propylene glycol as a co-surfactant in triacetin solution, with poorly water-soluble active ingredients (indomethacin, sodium diclofenac and chloramphenicol) and water. The systems obtained were spontaneous microemulsions, stable for surfactant concentrations of 15% and propylene glycol concentrations of 40%, the active ingredient being present at a concentration of 0.5% (w/w) (Siebenbrodt I. e Keipert S., Poloxamer-Systems as Potential Ophthalmics - II. Microemulsions, Eur. J. Pharm. Biopharm., 39 (1993) 25-30).

It is right for their self-emulsifying features that microemulsions in the pharmaceutical field were later named "self micro-emulsifying drug delivery sytems" (**SMEDDS**), and with that acronym they later gained relevance in the scientific literature. Recently, the self-emulsifying systems (both "self-micro-emulsifying drug delivery systems" and "self-emulsifying drug delivery systems", **SEDDS**, that is self emulsifying systems with dispersed particles of size corresponding to the particle size of conventional emulsions, but obtained by addition of remarkable amounts of surfactants and co-surfactants rather than by energy input) have received an enormous attention after the first marketing of a new pharmaceutical preparation for oral administration based on cyclosporine A, Neoral ® (Novartis). This product corresponds to the original product Sandimmun®, but has been re-formulated with the introduction of a self-emulsifying delivery system, which remarkably improved its bioavailability.

As noted, the self-emulsifying delivery systems, both as microemulsions (SMEDDS) and as emulsions with dispersed phase droplets of bigger size, obtained by chemical route instead of energy input (SEDDS), have the clear advantage of forming spontaneously, and remaining stable for an indefinite time, and they can also improve the bioavailability of the drugs carried by them. However, they seem to be unsuitable to the ophthalmic field, as they require the use of remarkable amounts of surfactants. Specially in the therapy of chronic pathologies, such as glaucoma or age-related macular degeneration, the continuous administration of surfactants on the ocular surface, even if chosen from the least aggressive ones, may bring to quite important side effects on the corneal surface. Actually, it has already been observed that in the ophthalmological field microemulsions could result in ocular toxicity risks, owing to their excessive surfactant concentrations (T.F. Vandamme, Microemulsions as ocular drug delivery systems: recent developements and future challenges, Prog. Retin. Eye Res. 21 (2002) 15-34).

Several examples of SMEDDS systems proposed in the scientific literature demonstrate that these systems, although being stable, capable of overcoming the problem of a poor water-solubility of the drug and of increasing the bioavailability thereof upon oral administration, always contain extremely high concentrations of surfactants, generally not less than 30% by weight of the overall formulation. (Wu W., et al., Enhanced bioavailability of silymarin by self-microemulsifying drug delivery system, Eur J. Pharm. Biopharm., 63 (2006) 28-294; Bachhav Y.G e Patravale V.B., AAPS PharmSci-Tech., 10: 2 (2009) 482-47). Also the presence of the co-surfactant, the lipophilic solubilizing agent and, sometimes, of the alcoholic solvent involves further possible tolerability problems for the topical ophthalmic administration. All of such products, although being pharmaceutically acceptable for oral administration, may have a chemical nature unsuitable to the administration on the ocular surface.

The foregoing reasons explain why at present the self-emulsifying administration systems have found wide room in works focused on oral administration, and therefore finalized to a gastrointestinal delivery of active ingredients, but not in works in the ophthalmic pharmaceutical field. As a matter of fact, no scientific works dealing with the use of SMEDDS systems for the topical ophthalmic delivery of lipophilic or scarcely water-soluble active ingredients are known to date.

In the light of the foregoing, the present invention is aimed at formulating lipophilic or scarcely soluble active ingredients, having therapeutic indications in ophthalmology, in microemulsions or SMEDDS systems which may stably carry the active ingredient while increasing, if possible, its bioavailability. In spite of being made of oil-in-water emulsions, such preparations must be endowed with the physical and chemical stability required as a necessary condition for a pharmaceutical product, so as to allow the storage of the product for the time required in such case, preferably also in non-refrigerated conditions.

According to the invention, it has been found that using a particular combination of oily phase and emulsifying agent/surfactant it is possible to obtain self-emulsifying delivery systems of the SMEDDS type, which are able to form practically spontaneously, and are thermodynamically stable, but differently from the conventional SMEDDS systems have very low concentrations of emulsifying agent. Further, differently from the conventional SMEDDS systems, the systems proposed according to the invention do not require a co-surfactant in the formulation, nor a particular solvent which could be incompatible with the direct administration on the ocular surface.

The oily phase of the combination of ingredients proposed according to the invention consists of medium chain triglycerides (MCT), already cited in connection with the prior art and used since long time in the ophthalmic field. According to the official pharmacopoeias, MCTs consist of natural mixtures of fatty acids with chains of 6-12 carbon atoms. Examples of MCT products on the market, already used in the ophthalmic field, are Miglyol® (Dynamit Nobel, SE) and TCM® (Societé des Oleagineux, FR).

The agent having functions of surfactant/emulsifier in the combination of ingredients proposed according to the invention is a vitamin E derivative (specifically, a derivative of the most widespread component of vitamin E, α-tocopherol) containing a hydrophilic polar head formed by a polyethylene glycol chain linked to the tocopherol structure by means of the diester bridge of succinic acid. The compound, the chemical name of which is d-alpha-tocopheryl polyethylene glycol 1000 succinate (wherein 1000 is referred to the length of the PEG chain), is obtained by esterification of PEG 1000 with d-α-tocopheryl succinate and is also known with names TPGS (English acronym) and vitamin E TPGS. Starting from the '50s the said compound has been used as surfactant and emulsifier specially in pharmaceutical compositions, also with the function of promoting absorption and improving the bioavailability of vitamin E preparations.

Vitamin E TPGS is also described in documents more closely related to the ophthalmic field, in particular, the patent US 5886030 (Maniar, assigned to Alcon Laboratories Inc.), which proposes the use of TPGS in ophthalmic composition with non-steroidal anti-inflammatory active ingredients (NSAIDs), with the function of reducing the irritating effect of NSAIDs when applied directly on the cornea, and also o ameliorating the active ingredient solubility. It is to be noted that in this case the compositions concerned are not emulsions, rather they are true solutions, where the solubility of the active ingredient (NSAID) is increased by the presence of TPGS. The concentration proposed for TPGS is comprised between 0.1 and 20% by weight.

The use of TPGS in ophthalmic formulations is also proposed in the document US2009/0092665 (Mitra et al.), where TPGS is used together with another surfactant (PEG alkyl ether or PEG alkyl aryl ether) to form micellar solutions for carrying calcineurine inhibitors active ingredients, in particular cyclosporine A in a topical ophthalmic preparation. The concentration proposed for TPGS is comprised between 0.01 and 20% by weight, and also in this case, the ophthalmic preparations proposed are not self-emulsifying systems.

According to the present invention it has been found, on the other hand, that using very low concentrations of the surfactant/emulsifier vitamin E TPGS in combination with equally low concentrations of MCT oils as the oily carrier and applying a mild magnetic stirring to the mixture, self-emulsifying delivery systems are obtained which are surprisingly stable and provided with all the features that a SMEDDS system should have. In particular, the preparation so obtained appears to have the clear aspect typical of microemulsions, and an average size of the dispersed phase below 100 nm.

It has also been found that the self-emulsifying system proposed according to the invention, in addition to optimally solving the problem of carrying lipophilic active ingredients in ophthalmic preparations, thanks to their stability and to their further characteristics already cited, can be advantageously used as tear substitutes (artificial tears).

As a matter of fact, the precorneal residence time of a tear substitute is essential in order to maintain the cornea and the tissues connected to the ocular surface healthy and free from irritation. Considering that the tear film is formed by the following three layers:
a) a mucous layer formed by mucins, directly in contact with corneal epithelium,
b) an intermediate aqueous layer rich in biopolymers and micronutrients (such as ions and mineral salts),
c) an exterior lipid layer, directly in contact with the environment and rich in triglycerides and natural waxes and phospholipids,
and considering that the ocular comfort and the correct functioning of the lachrymal apparatus depend on the integrity and equilibrium of all of the three cited layers, the ophthalmic preparation proposed according to the invention shows the suitable features for a valid use as a tear substitute.

Therefore, the present invention specifically provides a topical ophthalmic preparation consisting of an oil-in-water microemulsion comprising:
one or more liposoluble active ingredients;
an emulsifying agent consisting of d-α-tocopheryl polyethylene glycol 1000 succinate (TPGS);
an oily component consisting of medium chain triglycerides (MCT);
an ophthalmologically acceptable aqueous phase;
wherein the concentration of TPGS is comprised between 0.1 and 5% by weight and the weight ratio of MCT to TPGS is comprised between 1:2.8 and 1:3.6, the average size of the dispersed particles of oil phase being not greater than 100 nm, and wherein no co-surfactants are present.

More specifically, it has been found that for the use as carriers for several lipophilic active ingredients, such as, e.g., steroids as difluprednate, or prostaglandin derivatives as latanoprost or cyclosporine A, the optimal ratio of MCT to TPGS is 1:3.33.

In this case, according to the invention, with extremely low concentrations of surfactant, in the range of 1-2% by weight, and even lower concentrations of medium chain triglycerides, in the range of 0.25%-1% by weight, microemulsions of the said active ingredients in aqueous phase are obtained which are formed almost spontaneously and show all of the features of the SMEDDS systems, including a practically unlimited stability. The fact that these systems are microemulsions is also confirmed by the average size of the dispersed particles of the oily phase, which, in the cited cases, is lower than 20 nm and often does not exceed 10 nm.

The delivery systems according to the invention are also characterized in that they do not contain other additional components besides the couple oily component-surfactant, which are necessary to the formation and maintenance of the microemulsion. Additional components, such as the co-surfactant, or various solvents in the aqueous or oily phase, may be undesired for an ophthalmic preparation. As noted in the foregoing, the prior art products (SMEDDS) often contain solvents, such as, e.g., ethanol and other alcohol compounds, which are not suitable for topical ophthalmic administration, or which could even be aggressive to the ocular mucous membrane. In the present case the oily phase may consist of the only three components cited, and the aqueous phase contains a water-soluble component which is well tolerated by the ocular mucosa, preferably glycerol, and optionally the usual additives typical of the aqueous eye-drops.

According to some preferred embodiments, therefore, the ophthalmic preparation according to the invention comprises from 0.002% to 5% by weight of liposoluble active ingredient, in particular an active ingredient selected from the group consisting of: steroidal anti-inflammatory drugs (such as difluprednate, triamcinolone acetonide, dexamethasone isonicotinate, dexamethasone palmitate, dexamethasone acetate, prednisolone acetate, methyl-prednisolone, beclomethasone, beclomethasone dipropionate, anacortive acetate, fluocinolone), non-steroidal anti-inflammatory drugs (such as diclofenac, ketoprofen, ibuprofen, sodium naproxene, acetyl salicylic acid, nimesulide, flurbiprofen, indometacin, sulindac, meclofenamate, piroxicam, ketorolac, celecoxib), prostaglandin derivatives (such as latanoprost, bimatoprost, travoprost and unoprostone), calcineurin inhibitors or immunosuppressants (such as cyclosporine A), antioxidants (such as, e.g., carotenoids, α-, β-, γ-, δ-tocopherol and α-, β-, γ-, δ-tocotrienol and derivatives thereof, ascorbic acid and salts and esters thereof, retinoic acid, retinol and esters thereof, ubiquinol, ubiquinone, reduced glutathione, flavonoids, lipoic acid), active ingredients of a biotechnological origin (such, as e.g., etanercept, pegaptanib, ranibizumab, bevacizumab).

The proposed preparation may comprise, finally, other components required or preferred for a topical ophthalmic formulation, such as a buffer (preferably, but not exclusively, phosphate buffer), one or more osmotizing agents, and one or more antimicrobial agents.

As concerns the use of the proposed self-emulsifying system as artificial tears, the tear substitutes presently in use for the treatment of dry eye syndrome are not able, generally, to properly and effectively maintain the physico-chemical and biological characteristics of the three layers of the tear. Actually, said products are either formulated with the aim of nourishing and protecting the only mucous component of the tear film (such as the products based on hyaluronic acid or of cellulose derivatives), or they only integrate the outer lipid layer of the tear fluid, aiming at lubricating and limiting the evaporation of the tear fluid (as the preparations based on lipid ingredients).

Therefore, the present invention further specifically provides an ophthalmic preparation for use as artificial tears consisting of an oil-in-water emulsion comprising:
an emulsifying agent consisting of d-α-tocopheryl polyethylene glycol 1000 succinate (TPGS);
an oily component consisting of medium chain triglycerides (MCT);
an ophthalmologically acceptable aqueous phase containing a polysaccharide polymer or a cellulose derivative selected from the compounds used for the formulation of artificial tears, wherein the concentration of TPGS is comprised between 0.01 and 10% by weight and the weight ratio of MCT to TPGS is comprised between 1:1 and 1:4, the average size of the dispersed particles of oily phase being not higher than 100 nm, and wherein no co-surfactants are present.

The artificial tear preparation according to the invention is able to act simultaneously on the three layers of the tear film, nourishing, hydrating and protecting the lipid layer of the tear thanks to the presence of the medium chain triglycerides (MCT), the aqueous tear layer thanks to the presence of hydrophilic and osmoprotective substances as glycerol or threalose, sodium chloride and/or sodium phosphate, and the mucous layer thanks to the possibility of adding a polysaccharide polymer such as hyaluronic acid, xanthan gum or one or more cellulose derivatives, such as carboxymethyl cellulose and the like, to the formulation. In such a way, the preparation according to the invention advantageously provides to carrying on the ocular surface a polymer and mineral salts essential to the homeostasis of the tear film. Indeed, it is widely demonstrated that the combination of polymers such as hyaluronic acid and salts such as sodium chloride and phosphates, by stiffening or breaking the interfacial film between oil and water, bring about physical instability phenomena in the emulsion systems.

The preparation of the microemulsions according to the invention appears to be particularly simple and, as it is typical for the SMEDDS systems, it does not require any energy input, either in terms of heating or in terms of stirring or homogeneization. The components of the oily phase (MCT, vitamin E TPGS and the active ingredient, when present) are weighed, placed together in a vessel and mechanically stirred until a clear and slightly viscous solution is obtained. The components of the oily phase (preferably glycerol, together with the possible further water-soluble ingredients including, in the case of artificial tears, one or more of the compounds of the polysaccharide type already known and used in this field) are weighed and dissolved in water until a clear and very fluid solution is obtained. The two phases are then mixed and gently stirred with a magnet. During the gentle stirring the formation of small aggregates having the consistency of a gel is noted. The latter rapidly dissolve, leaving a clear and fluid liquid product. The process thus brings to the formation of a microemulsion, the features if which will be illustrated in detail in the following experimental section. Finally, the pH is measured and adjusted to values comprised in the range between 5.5 and 7.2, preferably using phosphate buffer. The microemulsion obtained is then sterilized by filtration through 0.22 µm filters. As noted, all of the operations needed for the formation of the microemulsion are carried out at room temperature.

Thus, according to a further aspect thereof, the present invention concerns a process for the production of a topic ophthalmic preparation in microemulsion as previously defined, which process comprises the following operations:
i) mixing together fixed amounts of one or more liposoluble active ingredients (when present), TPGS and MCT, until a clear oily solution is obtained;
ii) mixing together fixed amounts of the components of the said aqueous phase;
iii) joining together said oily solution and said aqueous solution, mixing with a magnetic mixer until a transparent and fluid microemulsion is obtained;
iv) measuring the pH of the said microemulsion and adjusting it to a fixed value comprised between 5.5 and 7.2, by addition of a buffer,
all of the above operations being carried out at room temperature.

The specific features of the invention, as well as the advantages thereof and its operating modes, will be more evident with reference to the detailed description presented for merely exemplificative purposes in the following, together with the results of the experimentation carried out on it and with the comparative data with the prior art.

The examples of preparations described below are produced by applying the following procedure. The components of the oily phase (MCTs, vitamin E TPGS and the various active ingredients) are weighed, placed in a glass vial in a water bathe at 30°C and then magnetically stirred until a clear, slightly viscous solution is obtained. The component of the aqueous phase (glycerin) is weighed and dissolved in water until a clear and very fluid solution is obtained. The two phases are then mixed and gently stirred with a magnet. The small aggregates formed, of a gel consistence, are rapidly dissolved, leaving a clear and fluid liquid product. The pH is measured and adjusted to values comprised between 5.5 and 7.2 using a phosphate buffer. The microemulsion obtained is then sterilized by filtration through 0.22 µm filters.

### EXAMPLE 1

### Difluprednate formulation in ophthalmic microemulsion

**MDV 0911 DIFLU**

| | |
|---|---|
| MCT | 0.7% |
| vitamin E TPGS | 2% |
| difluprednate | 0.05% |
| glycerin | 2% |
| disodium phosphate | 0.02% |
| water | q.s. to 100 ml |

By following the same formulation procedure also other steroids used in the ophthalmic field have been formulated, in particular triamcinolone acetonide, dexamethasone isonicotinate, dexamethasone palmitate, dexamethasone acetate, prednisolone acetate, methyl-prednisolone, beclometasone, beclometasone dipropionate, anacortive acetate, fluocinolone. The concentrations used for the various components are reported below.

| | |
|---|---|
| MCT | 0.6-0.7% |
| vitamin E TPGS | 2% |
| steroide | 0.05 - 0.1% |
| glycerol | 2% |
| disodium phosphate | 0.02% |
| water | q.s. to 100 ml |

### EXAMPLE 2

### Latanoprost formulation in ophthalmic microemulsion

By using the technology of the system MDV 0911 according to the invention a stable latanoprost composition has been produced.

**MDV 0911 LAT**

| | |
|---|---|
| MCT | 0.6% |
| vitamin E TPGS | 2% |
| latanoprost | 0.005% |
| glycerol | 2% |
| disodium phosphate | 0.02% |
| water | q.s. to 100 ml |

As it is known, Xalatan® (Pfizer) is an aqueous solution containing 0.005% latanoprost. Such compound, which is the isopropyl ester of an analog of prostaglandin F_{2α} (PGF_{2α}), is used in ophthalmology for the treatment of ocular hypertension and open angle glaucoma.

It is known that the chemical instability of this molecule in the formulation present in the Xalatan product results in the need to store the drug in refrigerated conditions. In the formulation proposed according to the invention, on the contrary, latanoprost is solubilized within a microemulsion system, ,hich makes it remarkably more stable in comparison with the aqueous formulation of the prior art. Actually, after accelerated stability tests carried out at 70°C, the results of which are synthesized in the following Table 1, the latanoprost present in the preparation named **MDV 0911 LAT** did not show the presence of any degradation product, differently from the commercial product Xalatan.

**TABLE 1**

| Accelerated stability test at 70°C. Percent of latanoprost present in the Xalatan® composition and present in MDV 0911 LAT | | | | |
|---|---|---|---|---|
| **Product** | **3 DAYS** | **5 DAYS** | **7 DAYS** | **15 DAYS** |
| Xalatan | 80% | 34% | n.d. | n.d. |
| LAT | 100% | 100% | 100% | 100% |

| | | | | |
|---|---|---|---|---|
| n.d.: not detectable | | | | |

This feature affords, for lantanoprost formulated according to the invention, the possibility of a long storage at room temperature, thus avoiding the cold chain (industrial advantage) and the storage in refrigerator (advantage for the user).

In a similar way other prostaglandine derivatives have been formulated, namely bimatoprost, travoprost e unoprostone. The concentrations used are reported below.

| | |
|---|---|
| MCT | 0.6% |
| vitamin E TPGS | 2% |
| prostaglandin derivative | 0.005% |
| glycerol | 2% |
| disodium phosphate | 0.02% |
| water | q.s. to 100 ml |

### EXAMPLE 3

### Cyclosporine A formulation in ophthalmic microemulsion

The MDV 0911 system has also been used for carrying the immuno-suppressant agent cyclosporine A, by following the same formulation procedure described in the foregoing.

**MDV 0911 CYCLO**

| | |
|---|---|
| MCT | 0.6% |
| vitamin E TPGS | 2% |
| cyclosporine A | 0.05%-0.% |
| glycerol | 2% |
| disodium phosphate | 0.02% |
| water | q.s. to 100 ml |

The preparation in microemulsion obtained according to the teachings of the invention has been compared with other preparations having the same fundamental components (MCT and TPGS) but in proportions different from the proportions specified according to the invention, as well as with the already marketed product of the prior art. The quali-quantitative compositions of the preparations in comparison are specified in the following table.

| **Component** | **MDV0911 CYCLO** | **MDV0911 CYCLO2** | **MDV0911 CYCLO3** | **RESTASIS®** |
|---|---|---|---|---|
| cyclosporine A | 0.05% | 0.05% | 0.05% | 0.05% |
| MCT | 0.6% | 1% | 1% | -- |
| vit. E TPGS | 2% | 0.2% | 0.3% | -- |
| castor oil | -- | -- | -- | 1.25% |
| Tween 80 | -- | -- | -- | 1% |
| Pemulen | -- | -- | -- | 0.05% |
| glycerol | 2% | 2% | 2% | 2.2% |
| disodium phosphate | 0.02% | 0.02% | 0.02% | |
| water | q.s. to 100 ml | q.s. to 100 ml | q.s. to 100 ml | q.s. to 100 ml |

The physical stability of the systems in comparison has been followed by measuring the sizes of the dispersed globules (nm). The latter has been assessed by means of a quasi-elastic light scattering after dilution in water of the sample prepared, using a High Performance Particle Sizer (Malvern Instrument, UK).

The results obtained are reported in the following Table 2.

**TABLE 2**

| Physical stability of systems containing cyclosporine A at the time zero and after 2 and 3 months. Storage at 40°C | | | |
|---|---|---|---|
| **Time (mohts)** | **0** | **3** | **6** |
| **System** | **size (nm)** | | |
| CYCLO | 15 | 14 | 15 |
| CYCLO2 | phase separation | -- | -- |
| CYCLO3 | phase separation | -- | -- |
| RESTASIS® | 279 | 514 (56%) 267 (43%) | phase separation |

The previous results demonstrate that if compositions with the couple MCT/vitamin E TPGS are produced out of the concentrations range according to the invention physically instable systems are obtained. Further, if compared with composition present on the market containing 0.005% cyclosporine A, the system proposed succeeded in providing dispersed globules of lower size and much more stable.

### EXAMPLE 4

### Diclofenac formulation ophthalmic microemulsion

The preparation technology of the self emulsifying system according to the invention has been used to obtain a topical ophthalmic preparation based on diclofenac..

**MDV 0911 DICLO**

| | |
|---|---|
| MCT | 0.6% |
| vitamin E TPGS | 2% |
| diclofenac | 0.1% |
| glycerol | 2% |
| disodium phosphate | 0.02% |
| water | q.s. to 100 ml |

In a similar way other non-steroidal anti-inflammatory active ingredients have been formulated, such as, e.g.: ketoprofene, ibuprofene, sodium naproxsene, acetylsalicylic acid, nimesulide, flurbiprofene, indometacin, sulindac, meclofenamate, piroxicam, ketorolac, celecoxib.

In this case, the remarkable advantages of the proposed formulation according to the invention resides in the possibility of carrying the said active ingredients at neutral pH, thus remarkably improving the compliance by the patient and probably the pharmacokinetics of the formulated active ingredients.

### EXAMPLE 5

### Antioxidants formulation in ophthalmic microemulsion

By using the delivery system according to the invention it is also possible to formulate molecules with antioxidant activity of a lipophilic and non-lipophilic nature, as well as their pro-drugs and their mixtures, The most important examples of antioxidant molecules, used also in the ophthalmic field, are carotenoids, beta-carotene, lutein, zeaxantin, licopene, the compounds derived from vitamin E (α-, β-, γ-, δ-tocopherol e α-, β-, γ-, δ-tocotrienol) and their derivatives, ascorbic acid and its salt and esters (e.g., the ascorbil palmitate), retinoic acid, retinol and its esters (such as retinyl palmitate), ubiquinol and ubiquinone (CoQ₁₀), reduced glutathione, flavonoids (antocyans, calcones, flavanones, flavones and isoflavones), lipoic acid.

The quail-quantitative composition of a antioxidant preparation in ophthalmic emulsions according to the invention is reported below.

| | |
|---|---|
| MCT | 0.6% |
| vitamin E TPGS | 2% |
| antioxidant | 0.05 - 2% |
| glycerol | 2% |
| disodium phosphate | 0.02% |
| water | q.s. to 100 ml |

The proposed formulation allowed to remarkably increase the chemical stability and topical tolerability of the antioxidant active ingredients cited.

### EXAMPLE 6

Formulation of biotechnological active ingredients in ophthalmic microemulsion

The system according to the invention has also be used for formulating molecules of a biotechnological nature , such as, e.g. etanercept, pegaptanib, ranibizumab, bevacizumab. The quali-quantitative compositions of two preparation according to the invention are reposted below.

| | |
|---|---|
| MCT | 0,3% |
| vitamin E TPGS | 1% |
| etanercept | 0.05% |
| glycerol | 2% |
| disodium phosphate | 0.02% |
| water | q.s. to 100 ml |

| | |
|---|---|
| MCT | 0.6% |
| vitamin E TPGS | 2% |
| pegaptanib | 2% |
| glycerol | 2% |
| disodium phosphate | 0.02% |
| water | q.s. to 100 ml |

### Characterization and stability of self-emulsifying systems in microemulsion according to the invention

The stability of the microemulsified systems has been followed from a physical point of view measuring the size of the dispersed globules (nm) and the polidispersity index (Poly)

The size of the dispersed globules of the various system obtained has been measured by a quasi-elastic light scattering after the dilution in water of the sample prepared using High Performance Particle Sizer (Malvern Instrument, UK). This instrument has also been employed to assess the polydidpersity index.

**TABLE 3**

| Light scattering and polydispersity of MDV 0911 systemS of examples **1-4.**Storage: 40°C | | | | |
|---|---|---|---|---|
| **Time (months)** | **0** | **1** | **3** | **6** |
| **System** | **size (nm)** | | | |
| DIFLU | 10 | 10 | 12 | 11 |
| LAT | 8 | 8 | 9 | 8 |
| CYCLO | 15 | 12 | 14 | 15 |
| DICLO | 10 | 10 | 11 | 12 |
| | | | | |
| Poly T zero | 0,096 | 0.145 | 0.132 | 0.155 |

For the evaluation of the chemical stability, the percent concentration of the active ingredients present in the microemulsions in conditions of accelerated stability at 40°C, according to the ICH standard, has been followed by means of HPLC.

The experimental results obtained are reported in the following Table.

**TABLE 4**

| Percent of active ingredient respect to time 0, after 3 and 6 months for the MDV 0911 systems system of examples **1-4.** Storage: 40°C | | | | |
|---|---|---|---|---|
| **Time (months)** | **0** | **1** | **3** | **6** |
| **System** | **(%)** | | | |
| DIFLU | 100 | 99 | 98 | 98 |
| LAT | 100 | 100 | 100 | 100 |
| CYCLO | 100 | 100 | 97 | 97 |
| DICLO | 100 | 98 | 98 | 97 |

As it may be observed from the foregoing experimental data, couple MCT/vitamin E TPGS at the concentrations and in the ratios used, results in self-emulsifying systems (microemulsions or SMEDDS) which are remarkably stable (40°C for six months) not only from the physical stand point but also from the chemical stand point. Indeed , such systems are able to protect from the degradation phenomena molecules for which of the storage in refrigerator is presently prescribed, owing to their instability.

From the foregoing description it appears that the ophthalmic preparation in microemulsion proposed according to the invention has the ability of forming spontaneously, thus behaving as a SMEDDS system, but it is obtained with a remarkably low concentration of surfactant and without the aid of organic solvents which may be aggressive to the ocular mucous. The system according to the invention is capable of stably carrying higly lipophilic active ingredients using an oli/surfactant couple, MCT/TPGS in extremely low concentration. The system has, thus, all the necessary requirements for an ophthalmic administration effective and free from side effects.

## Claims

1. A topical ophthalmic preparation consisting of an oil-in-water microemulsion comprising:
one or more liposoluble active ingredients;
an emulsifying agent consisting of d-α-tocopheryl polyethylene glycol 1000 succinate (TPGS);
an oily component consisting of medium chain triglycerides (MCT);
an ophthalmologically acceptable aqueous phase;
wherein the concentration of TPGS is comprised between 0.1 and 5% by weight and the weight ratio of MCT to TPGS is comprised between 1:2.8 and 1:3.6, the average size of the dispersed particles of oily phase being not greater than 100 nm, and wherein no co-surfactants are present.

2. An ophthalmic preparation according to claim 1, wherein the said ratio is 1:3.33 and the said average size of the particles of oily phase is not greater than 20 nm.

3. An ophthalmic preparation according to claim 2, wherein the concentration of TPGS is comprised between 1 and 2% by weight and the concentration of MCT is comprised between 0.25% and 1% by weight.

4. An ophthalmic preparation according to any one of claims 1-3, containing from 0.002% to 5% by weight of the liposoluble active ingredient.

5. An ophthalmic preparation according to claim 4, wherein the said liposoluble active ingredient is selected from the group consisting of: steroidal anti-inflammatory drugs, non-steroidal anti-inflammatory drugs, prostaglandin derivatives selected from latanoprost, bimatoprost, travoprost and unoprostone, calcineurin inhibitors or immunosuppressants, antioxidants, active ingredients of a biotechnological origin.

6. An ophthalmic preparation according to claim 5, wherein the said ophthalmologically acceptable aqueous phase consists of an aqueous solution of glycerol.

7. An ophthalmic preparation according to claims 5 or 6, also comprising a buffer.

8. An ophthalmic preparation according to any one of claims 6 or 7, also comprising one or more osmotizing agents.

9. An ophthalmic preparation for use as artificial tears consisting of an oil-in-water emulsion comprising:
an emulsifying agent consisting of d-α-tocopheryl polyethylene glycol 1000 succinate (TPGS);
an oily component consisting of medium chain triglycerides (MCT);
an ophthalmologically acceptable aqueous phase containing a polysaccharide polymer or a cellulose derivative selected from the compounds used for the formulation of artificial tears, wherein the concentration of TPGS is comprised between 0.01 and 10% by weight and the weight ratio of MCT to TPGS is comprised between 1:1 and 1:4, the average size of the dispersed particles of oily phase being not higher than 100 nm, and wherein no co-surfactants are present.

10. A process for the production of a topical ophthalmic preparation in microemulsion as defined in claim 1, comprising the following operations:
i) mixing together fixed amounts of one or more liposoluble active ingredients, TPGS and MCT, until a clear oily solution is obtained;
ii) mixing together fixed amounts of the components of the said aqueous phase;
iii) joining together said oily solution and said aqueous solution until a clear and fluid microemulsion is obtained;
iv) measuring the pH of the said microemulsion and adjusting it to a fixed value comprised between 5.5 and 7.2, with addition of a buffer,
all of the above operations being carried out at room temperature.

## Patentansprüche

1. Topisches Augenheilmittel, das aus einer ÖI-in-Wasser-Mikroemulsion besteht, umfassend:
einen oder mehrere fettlösliche Wirkstoffe;
einen Emulgator, der aus d-α-Tocopheryl-Polyethylen-Glycol-1000-Succinat (TPGS) besteht;
eine ölige Komponente, die aus mittelkettigen Triglyceriden (MCT) besteht;
eine ophthalmologisch zulässige wässrige Phase;
wobei die Konzentration von TPGS zwischen 0,1 und 5 Gew.-% beträgt und das Gewichtsverhältnis von MCT zu TPGS zwischen 1:2,8 und 1:3,6 beträgt, wobei die durchschnittliche Größe der feinverteilten Partikel öliger Phase nicht größer als 100 nm ist und wobei keine Co-Tenside vorliegen.

2. Augenheilmittel nach Anspruch 1, wobei das Verhältnis 1:3,33 beträgt und die durchschnittliche Größe der Partikel öliger Phase nicht größer als 20 nm ist.

3. Augenheilmittel nach Anspruch 2, wobei die Konzentration von TPGS zwischen 1 und 2 Gew.-% beträgt und die Konzentration von MCT zwischen 0,25 und 1 Gew.-% beträgt.

4. Augenheilmittel nach einem der Ansprüche 1 bis 3, das von 0,002 bis 5 Gew.-% des fettlöslichen Wirkstoffs enthält.

5. Augenheilmittel nach Anspruch 4, wobei der fettlösliche Wirkstoff aus der Gruppe ausgewählt ist, die aus Folgenden besteht: steroide entzündungshemmende Mittel, nichtsteroide entzündungshemmende Mittel, Prostaglandin-Derivate, die aus Latanoprost, Bimatoprost, Travoprost und Unoproston ausgewählt sind, Calcineurininhibitoren oder Immunsuppressiva, Antioxidanzien, Wirkstoffe biotechnologischen Ursprungs.

6. Augenheilmittel nach Anspruch 5, wobei die ophthalmologisch zulässige wässrige Phase aus einer wässrigen Lösung von Glycerol besteht.

7. Augenheilmittel nach Anspruch 5 oder 6, das auch einen Puffer umfasst.

8. Augenheilmittel nach einem der Ansprüche 6 oder 7, das auch ein oder mehrere Osmosemittel umfasst.

9. Augenheilmittel zur Verwendung als künstliche Tränen, das aus einer ÖI-in-Wasser-Emulsion besteht, umfassend:
einen Emulgator, der aus d-α-Tocopheryl-Polyethylen-Glycol-1000-Succinat (TPGS) besteht;
eine ölige Komponente, die aus mittelkettigen Triglyceriden (MCT) besteht;
eine ophthalmologisch zulässige wässrige Phase, die ein Polysaccharid-Polymer oder ein Zellulose-Derivat enthält, die aus den Verbindungen ausgewählt sind, die zur Rezeptierung künstlicher Tränen verwendet werden,
wobei die Konzentration von TPGS zwischen 0,01 und 10 Gew.-% beträgt und das Gewichtsverhältnis von MCT zu TPGS zwischen 1:1 und 1:4 beträgt, wobei die durchschnittliche Größe der feinverteilten Partikel öliger Phase nicht höher als 100 nm ist und wobei keine Co-Tenside vorliegen.

10. Verfahren zur Erzeugung eines topischen Augenheilmittels in Mikroemulsion wie in Anspruch 1 definiert, umfassend die folgenden Arbeitsgänge:
i) Zusammenmischen festgelegter Mengen eines oder mehrerer fettlöslicher Wirkstoffe, TPGS und MCT, bis eine klare ölige Lösung erhalten wird;
ii) Zusammenmischen festgelegter Mengen der Komponenten der wässrigen Phase;
iii) Zusammenfügen der öligen Lösung und der wässrigen Lösung, bis eine klare und flüssige Mikroemulsion erhalten wird;
Messen des pH-Werts der Mikroemulsion und Anpassen derselben an einen festgelegten Wert, der zwischen 5,5 und 7,2 liegt, unter Zufügung eines Puffers,
wobei alle oben genannten Arbeitsgänge bei Raumtemperatur ausgeführt werden.

## Revendications

1. Préparation ophtalmique topique consistant en une microémulsion huile-dans-eau comprenant :
un ou plusieurs principes actifs liposolubles ;
un agent émulsifiant consistant en le succinate de d-α-tocophéryl polyéthylène glycol 1000 (TPGS) ;
un composant huileux consistant en des triglycérides de chaîne moyenne (MCT) ;
une phase aqueuse ophtalmologiquement acceptable ;
dans laquelle la concentration en TPGS est comprise entre 0,1 et 5 % en poids et le rapport en poids des MCT sur le TPGS est compris entre 1:2,8 et 1:3,6, la taille moyenne des particules dispersées de la phase huileuse n'étant pas supérieure à 100 nm, et dans laquelle pas de co-surfactant n'est présent.

2. Préparation ophtalmique selon la revendication 1, dans laquelle ledit rapport est de 1:3,33 et ladite taille moyenne des particules de la phase huileuse n'est pas supérieure à 20 nm.

3. Préparation ophtalmique selon la revendication 2, dans laquelle la concentration en TPGS est comprise entre 1 et 2 % en poids et la concentration en MCT est comprise entre 0,25 % et 1 % en poids.

4. Préparation ophtalmique selon l'une quelconque des revendications 1 à 3, contenant de 0,002 % à 5 % en poids du principe actif liposoluble.

5. Préparation ophtalmique selon la revendication 4, dans laquelle ledit principe actif liposoluble est sélectionné dans le groupe consistant en : les anti-inflammatoires stéroïdiens, les anti-inflammatoires non stéroïdiens, les dérivés de la prostaglandine sélectionnés parmi le latanoprost, le bimatoprost, le travoprost et l'unoprostone, les inhibiteurs de la calcineurine ou immunosuppresseurs, les antioxydants, les principes actifs d'origine biotechnologique.

6. Préparation ophtalmique selon la revendication 5, dans laquelle ladite phase aqueuse ophtalmologiquement acceptable consiste en une solution aqueuse de glycérol.

7. Préparation ophtalmique selon les revendications 5 ou 6, comprenant également un tampon.

8. Préparation ophtalmique selon l'une quelconque des revendications 6 ou 7, comprenant également un ou plusieurs agents d'osmotisation.

9. Préparation ophtalmique pour son utilisation en tant que larmes artificielles consistant en une émulsion huile-dans-eau comprenant :
un agent émulsifiant consistant en le succinate de d-α-tocophéryl polyéthylène glycol 1000 (TPGS) ;
un composant huileux consistant en des triglycérides de chaîne moyenne (MCT) ;
une phase aqueuse ophtalmologiquement acceptable contenant un polymère de polysaccharide ou un dérivé de cellulose sélectionné parmi les composés utilisés pour la formulation de larmes artificielles,
dans laquelle la concentration en TPGS est comprise entre 0,01 et 10 % en poids et le rapport en poids de MCT sur le TPGS est compris entre 1:1 et 1:4, la taille moyenne des particules dispersées de la phase huileuse n'étant pas supérieure à 100 nm, et dans laquelle pas de co-surfactant n'est présent.

10. Procédé de production d'une préparation ophtalmique topique en microémulsion selon la revendication 1, comprenant les opérations suivantes :
i) mélange ensemble de quantités fixes d'un ou plusieurs principes actifs liposolubles, de TPGS et de MCT, jusqu'à ce qu'une solution huileuse claire soit obtenue ;
ii) mélange ensemble de quantités fixes des composants de ladite phase aqueuse ;
iii) rassemblement de ladite solution huileuse et de ladite solution aqueuse jusqu'à ce qu'une microémulsion fluide soit obtenue ;
iv) mesure du pH de ladite microémulsion et ajustement de celui-ci à une valeur fixe comprise entre 5,5 et 7,2, avec l'ajout d'un tampon,
l'ensemble des opérations ci-dessus étant réalisées à température ambiante.
